# EUROPEAN PATENT APPLICATION

(11) **EP 1 475 109 A1**
(43) Date of publication of application: **10.11.2004**
(21) Application number: 04018238.8
(22) Date of filing: 13.10.2000
(51) Int. Cl.: A61L 27/22, A61L 27/46

(54) **Formulations for delivery of osteogenic proteins**

(30) Priority: 15.10.1999 US 159703; 29.02.2000 US 185734
(62) Divisional of application: 00972168.9
(71) Applicant: Genetics Institute, LLC, Cambridge, MA 02140 (US)
(72) Inventor: Li, Rebecca, Bedford MA 01730 (US); Seeherman, Howard, Cambridge MA 02138 (US); Wozney, John, Hudson MA 01749 (US)
(74) Representative: Ingham, Stephen H.

(57) **Abstract**

An injectable formulation is disclosed for delivery of osteogenic proteins. The formulation comprises a comprising a pharmaceutically acceptable admixture of an osteogenic protein; and a hemostatic gelatin foam paste. Formulations comprising osteogenic protein, hemostatic gelatin foam paste and tricalcium phosphate are also disclosed.

## Description

### RELATED APPLICATIONS

This application claims the benefit of U.S. Provisional Application No. 60/159,703 filed on October 15, 1999 and U.S. Provisional Application No. 60/185,734 filed on February 29, 2000.

### BACKGROUND OF THE INVENTION

The subject invention relates to the field of osteogenic proteins and pharmaceutical formulations thereof. More particularly, the subject invention involves injectable pharmaceutical formulations comprising gelatin for delivery of osteogenic proteins.

Osteogenic proteins are those proteins capable of inducing, or assisting in the induction of, cartilage and/or bone formation. Many such osteogenic proteins have in recent years been isolated and characterized, and some have been produced by recombinant methods. For example, so-called bone morphogenic proteins (BMP) have been isolated from demineralized bone tissue (see e.g. Urist US 4,455,256); a number of such BMP proteins have been produced by recombinant techniques (see e.g. Wang et al. US 4,877,864 and Wang et al. US 5,013,549); a family of transforming growth factors (TGF-α and TGF-β) has been identified as potentially useful in the treatment of bone disease (see e.g. Derynck et al., EP 154,434); a protein designated Vgr-1 has been found to be expressed at high levels in osteogenic cells (see Lyons et al. (1989) Proc. Nat'l. Acad. Sci. USA 86, 4554-4558); and proteins designated OP-1, COP-5 and COP-7 have purportedly shown bone inductive activity (see Oppermann, et al. U.S. 5,001,691).

Various formulations designed to deliver osteogenic proteins to a site where induction of bone formation is desired have been developed. For example, certain polymeric matrices such as acrylic ester polymer (Urist, US 4,526,909) and lactic acid polymer (Urist, US 4,563,489) have been utilized.

Brekke et al., United States Patents 4,186,448 and 5,133,755 describe methods of forming highly porous biodegradable materials composed of polymers of lactic acid ("OPLA").

Okada et al., US 4,652,441, US 4,711,782, US 4,917,893 and US 5,061,492 and Yamamoto et al., US 4,954,298 disclose a prolonged-release microcapsule comprising a polypeptide drug and a drug-retaining substance encapsulated in an inner aqueous layer surrounded by a polymer wall substance in an outer oil layer.

Yamazaki et al., Clin. Orthop. and Related Research, 234:240-249 (1988) disclose the use of implants comprising 1 mg of bone morphogenetic protein purified from bone and 5 mg of Plaster of Paris. United States Patent 4,645,503 discloses composites of hydroxyapatite and Plaster of Paris as bone implant materials.

Collagen matrices have also been used as delivery vehicles for osteogenic proteins (see e.g. Jeffries, U.S. 4,394,370).

### SUMMARY OF THE INVENTION

The present invention provides injectable formulations allowing percutaneous delivery of osteogenic proteins. In one embodiment, the invention comprises compositions comprising a pharmaceutically acceptable admixture of an osteogenic protein together with a formulation of hemostatic gelatin foam. The injectable formulations of the invention allow closed fractures to be treated without an open reduction procedure as is necessary with implantable devices. Hemostatic gelatin foam maintains an interconnecting macroporous network conducive to cell infiltration and angiogenesis. It also absorbs blood and may concentrate an enriched population of osteoprogenitor cells at the trauma site where rhBMP-2 is present to act on the cells. In another embodiment, the injectable formulation comprises osteogenic protein, hemostatic gelatin foam injectable paste formulation, together with tricalcium phosphate.

Gelfoam or TCP/Gelfoam injectable pastes enhance healing in defects. Retention of rhBMP-2 (and perhaps other bone inductive proteins) at the site is enhanced when delivered in Gelfoam or TCP/Gelfoam paste compared to delivery in buffer. Addition of TCP enhances retention of the rhBMP-2 at the local site.

The methods and compositions of the present invention are useful for the preparation of formulations of osteoinductive proteins which can be used, among other uses, to promote the formation of cartilage and/or bone, for repair of tissue damage and fractures. The invention further provides methods for treating patients in need of cartilage and/or bone repair and/or growth.

### BRIEF DESCRIPTION OF THE DRAWINGS

Figure 1 sets forth the effect of 6 mLs Gelfoam in MFR00842+200µL BMP (hot+cold).
Figures 2 and 3 compares retention of I¹²⁵-rhBMP-2 post injection in the Rabbit Osteotony Model compared to rhBMP-2 delivered in a buffer.
Figures 4, 5, 6 and 7 set forth biomechanical and callus area assessment results in the rabbit osteotomy studies.

### DETAILED DESCRIPTION OF THE INVENTION

The present invention provides injectable compositions for the delivery of osteogenic proteins. The compositions comprise osteogenic protein and an injectable formulation comprising hemostatic gelatin foam. In other embodiments, the formulation includes the osteogenic protein, the formulation of hemostatic gelatin foam and tricalcium phosphate. The invention further provides a method for preparing the injectable gelatin foam and the invention includes the composition prepared by this composition of injectable gelatin foam.

Gelfoam formulations of the invention possess the advantages of (1) simple preparation procedure, (2) easy injectability through 18g and 20 g needles, (3) no additional crosslinkers, (4) long medical history as an implant material, (5) relatively low cost, and (6) no limit to working time (which may limit other materials that crosslink or "set"). One additional major advantage is that Gelfoam paste may be formulated and stored as a unitary product.

The osteogenic proteins useful with the fused sponges made in accordance with the subject invention are well known to those skilled in the art and include those discussed above. The preferred osteogenic proteins for use herein are those of the BMP class identified as BMP-1 through BMP-12 in US 4,877,864; US 5,013,649; WO 90/11366 published October 4, 1990; WO 91/18098 published November 28, 1991; WO 93/00432, published January 7, 1993; United States Serial Numbers 08/247,908 and 08/247,904, both filed May 20, 1994; and United States Serial Number 08/217,780, filed on March 25, 1994. The disclosure of the above publications are hereby incorporated by reference. The most preferred is BMP-2, the full length cDNA sequence of which is described in detail in the '649 patent. Of course, combinations of two or more of such osteogenic proteins may be used, as may fragments of such proteins that also exhibit osteogenic activity. Such osteogenic proteins are known to be homodimeric species, but also exhibit activity as mixed heterodimers. Heterodimeric forms of osteogenic proteins may also be used in the practice of the subject invention. BMP heterodimers are described in WO93/09229, the disclosure of which is hereby incorporated by reference. Recombinant proteins are preferred over naturally occurring isolated proteins. The amount of osteogenic protein useful herein is that amount effective to stimulate increased osteogenic. activity of infiltrating progenitor cells. and will depend upon the size and nature of defect being treated as well as the carrier being employed.

The formulations may be injected, for example into tendons, ligaments and/or their attachment site to bone. Injectable formulations may also find application to other bone sites such as bone cysts and closed fractures.

The dosage regimen will be determined by the clinical indication being addressed, as well as by various patient variables (e.g. weight, age, sex) and clinical presentation (e.g. extent of injury, site of injury, etc.). In general, the dosage of osteogenic protein will be in the range of from about 0.1 to 4 mg/ml.

The injectable osteogenic protein may be provided to the clinic as a single formulation, or the formulation may be provided as a multicomponent kit wherein, e.g. the osteogenic protein is provided in one vial and the porous particulate polymeric fused sponge is provided separately.

The formulations of the subject invention allow therapeutically effective amounts of osteoinductive protein to be delivered to an injury site where cartilage and/or bone formation is desired. The formulations may be used as a substitute for autologous bone graft in fresh and non-union fractures, spinal fusions, and bone defect repair in the orthopaedic field; in cranio/maxillofacial reconstructions; for prosthesis integration, especially as a surface coating to improve fixation of prosthetic implants such as hydroxyapatite coated prostheses; in osteomyelitis for bone regeneration; and in the dental field for augmentation of the alveolar ridge and periodontal defects and tooth extraction sockets. The methods and formulations of the present invention may be useful in the treatment and/or prevention of osteoporosis, or the treatment of osteoporotic or osteopenic bone. In another embodiment, formulations of the present invention may be used in the process known as distraction osteogenesis. When used to treat osteomyelitis or for bone repair with minimal infection, the osteogenic protein may be used in combination with porous microparticles and antibiotics, with the addition of protein sequestering agents such as alginate, cellulosics, especially carboxymethylcellulose, diluted using aqueous glycerol. The antibiotic is selected for its ability to decrease infection while having minimal adverse effects on bone formation. Preferred antibiotics for use in the devices of the present invention include vancomycin and gentamycin. The antibiotic may be in any pharmaceutically acceptable form, such as vancomycin HCl or gentamycin sulfate. The antibiotic is preferably present in a concentration of from about 0.1 mg/mL to about 10.0 mg/mL.] The traditional preparation of formulations in pharmaceutically acceptable form (i.e. pyrogen free, appropriate pH and isotonicity, sterility, etc.) is well within the skill in the art and is applicable to the formulations of the invention.

The following examples are illustrative of the present invention and are not limiting in any manner. Modifications, variations and minor enhancements are contemplated and are within the present invention.

### EXAMPLE 1

### RAT ECTOPIC STUDY

### A. Preparation of Hemostatic Gelatin Foam Matrix

Gelfoam® paste was formulated the day of implantation by hydrating one package (1g) of Gelfoam powder (Pharmacia/Upjohn) with 5 mL of MFR00842 buffer. Buffer was added directly to the sterile Gelfoam powder jar via pipet using aseptic technique in a biosafety cabinet. The resulting hydrated powder was then mixed with a sterile spatula for approximately two minutes until a cohesive homogeneous thick doughy paste consistency was achieved. Paste volume after hydration and mixing was approximately 6 mL. Formulation of 0.1 mg/mL rhBMP-2-containing paste was performed by addition of 142 µL rhBMP-2 bulk liquid added via sterile pipet evenly over the paste. The paste was then remixed to distribute the protein evenly through the entire volume. Final rhBMP-2 concentration in the paste was 0.1 mg/mL.

For injectable samples containing lyophilized FN, 5 mg lyophilized FN was dissolved in 0.5 mL supplied diluent. The 0.5 mL FN-containing volume was then added to the Gelfoam paste (entire 6 mL volume) and mixed with a sterile spatula prior to aliquoting into syringes. Final rhFN concentration was 770 µg/mL.

The Gelfoam paste was inserted into the back end of a 10 mL syringe using a sterile spatula. A sterile luer lock connector was then attached to the 10 mL syringe to facilitate loading of three, one-mL syringes with luer-lok (Becton Dickonson).

### B. Evaluation of rhBMP-2 Loading Reproducibility into Injectable Aliquots

The uniformity of rhBMP-2 loading into aliquots of Gelfoam paste (prepared as described above) was tested using radiolabeled I¹²⁵-rhBMP-2. In this evaluation, 40 mL of I¹²⁵-rhBMP-2 (0.5 mCi/mL) was mixed with 300 mL rhBMP-2 bulk liquid (cold protein). 200 mL of this protein solution (hot and cold rhBMP-2) was then added evenly to the 6 mL Gelfoam paste. The paste was then placed into a 10 mL syringe and then aliquoted into five one-mL syringes (labeled #1-5). This evaluation simulates a 30-fold dilution of rhBMP-2 solution in Gelfoam paste and the subsequent mixing and aliquoting procedure.

Simulating a typical injection procedure, four successive 100 mL samples were injected from each of the five syringes (18 g needles) into individual eppendorf tubes. The tubes were then placed in a gamma counter (Wallac 1470 Wizard Automatic Gamma Counter) for assessment of the I¹²⁵ counts per minute (CPM). Data was analyzed for the reproducibility of the mixing and aliquoting procedure.

### C. Surgical Procedure

The Gelfoam paste was tested for efficacy in the rat ectopic bone induction model. 4-5 week old male Long Evans rats were used in the study. Standard protocols were followed for animal care, implantation and explant analysis. After shaving, tagging, and weighing, animals were anesthetized by inhalation of Isoflurane in a bell jar.

Subcutaneous injections were performed in the ventral thoracic or scapular region. Intramuscular injections were performed in the quadriceps/calf muscle and repeated on the contralateral leg. For all injections an 18 g, 1 ½" needle was used. At the termination of the study (14 days), animals were euthanized by CO₂ inhalation. Post mortem, the devices were removed, weighed and examined for consistency (hardness), shape, presence of fluids, and vascularity. Explanted devices were then placed in labeled tissue cassettes and soaked in 70% ethanol.

### D. Histological Processing and Assessments

Specimens were received in 70% ethanol, inventoried and processed for undecalcified histology (Schenk, et al). Briefly, specimens were dehydrated with gradients of alcohol, cleared in xylene using Sakura VIP Tissue Processor. Specimens were infiltrated and embedded in methyl methacrylate, and allowed to polymerize for 3 - 5 days, at room temperature. Following complete polymerization, blocks were sectioned at 5 microns using a Riechert-Jung Polycut or Lecia 2065 Supercut. All slides were stained with a modified Goldner's Trichrome (Schenk et al). Stain results are: nuclei purple-black, cytoplasm and osteoid red, mineralized bone and collagen green, erythrocytes orange.

The size of the explant and the percentage of bone and residual matrix were calculated using histomorphometry techniques. Cellular response if present was noted as well as the type of response and presence or absence of inflammation.

Each slide was scored similarly for percent bone and residual matrix. Under the microscope, the percentage of bone and matrix was estimated and scored using the following scale.

| % Bone | Score |
|---|---|
| 0 (not observed) | 0 |
| <10 | 0.5 |
| 10-20 | 1 |
| 20-40 | 2 |
| 40-60 | 3 |
| 60-80 | 4 |
| 80-100 | 5 |

### E. Reproducibility of rhBMP-2-Gelfoam Paste Formulation

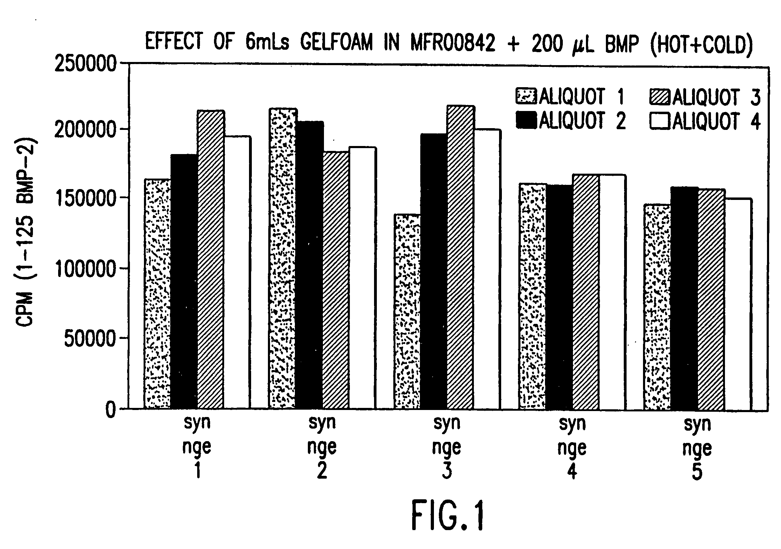

Standard descriptive statistics were computed for all outcome variables. The effect of rhBMP-2 treatment and addition of FN on ectopic bone formation was compared using an unpaired t-test. Tests were two-tailed and differences considered significant at p<0.05. Assessment of the overall mean of the 20 aliquots (5 syringes), showed a standard deviation of ± 13%. CPM per aliquot is shown in Figure 1. No apparent trend was observed as a function of aliquot number.

### G. Histological Evaluation

Control groups (no rhBMP-2): Histological observations showed that the matrix is resorbed within two weeks only when rhBMP-2 is present. Typical histology characteristics of the Gelfoam ectopic control implant after 14 days were a particulate structure, surrounded by giant cells and spindle cells at its periphery. Blood vessels are observed easily through the generally well-vascularized implants. Histology characteristics appeared to be similar among all controls, including Gelfoam alone, with Fibronectin, and implanted SQ or IM.

rhBMP-2 groups: Gelfoam/rhBMP-2-induced bone appeared to be formed mainly via the endochondral ossification path, since chondrocyte and cartilage matrix were consistently seen on the bone formation front, where the bone and the carrier matrix were separated. Some regions of bone formed through the direct mechanism were present. Bone formation with Gelfoam was initiated from the periphery and extended towards the center of the ectopic implant. Therefore a bony shell was formed at the early stage of the bone formation.

Significant differences were found between SQ and IM explants in all histology characterizations. Very little bone formation was observed in SQ explants while substantial bone formation was found in IM explants. In general, specimens in the IM site appeared to have a better overall response greater degree of matrix resorption and bone formation compared to the SQ specimens. Bone resorption is present in both IM and SQ specimens. IM specimens show significantly more mature bone, marrow and less matrix remaining. Minimal muscle degradation was noted. IM cellular infiltration of the carrier was greater than in the SQ specimens and consisted of numerous lymphocytes and giant cells.

Subcutaneous specimens displayed cellular infiltration of the matrix consisting of predominantly lymphocytes. Giant cells were abundant on the periphery of the material and a few noted towards the center. Matrix did not appear completely resorbed in many areas where SQ bone formation is present. Bone is formed adjacent to the carrier.

No significant difference could be found between implants formulated with Gelfoam alone and with Fibronectin, except Gelfoam alone has a slightly higher bone score. Both formulations performed poorly at the SQ site but very well at the IM site.

These observations are reflected in the average bone scores (Table 1) below.

**Table 1:**

| Histological Average Bone Scores | | | | | | | | | |
|---|---|---|---|---|---|---|---|---|---|
| Material/dose | Bone Score | | Matrix Score | | Implant Size (mm^2) | | Total Bone (mm^2) | | #n/ Group |
| | SQ | IM | SQ | IM | SQ | IM | SQ | IM | |
| Gelfoam | | | | | | | | | |
| 0 mg/mL | 0.00 | 0.00 | 5.00 | 4.88 | 29.19 | 32.37 | 0.00 | 0.00 | 8 |
| sd | 0.00 | 0.00 | 0.00 | 0.35 | 20.19 | 10.44 | 0.00 | 0.00 | |
| 0.1 mg/mL | 1.30 | 3.36 | 3.60 | 1.59 | 42.00 | 44.03 | 8.61 | 24.88 | 11 |
| sd | 1.01 | 0.74 | 1.26 | 0.66 | 9.26 | 16.10 | 8.40 | 12.87 | |

| Gelfoam + FN | | | | | | | | | |
|---|---|---|---|---|---|---|---|---|---|
| 0 mg/mL | 0.00 | 0.00 | 5.00 | 4.88 | 30.35 | 17.70 | 0.00 | 0.00 | 2 |
| sd | 0.00 | - | 0.00 | -- | 20.19 | 10.44 | 0.00 | 0.00 | |
| 0.1 mg/mL | 0.67 | 2.60 | 4.42 | 2.20 | 31.01 | 51.76 | 2.30 | 23.34 | 11 |
| sd | 0.68 | 0.89 | 0.80 | 1.10 | 12.57 | 24.93 | 2.48 | 15.39 | |

The injectable Gelfoam paste with rhBMP-2 induced mature bone within two weeks in both intramuscular and subcutaneous sites. Response in the intramuscular site was much more robust compared to the subcutaneous site. Large, well-vascularized bony explants were retrieved from the IM site. Average bone scores were 3.4 ± 0.7 for IM explants and 1.3 ± 1 for SQ explants containing rhBMP-2. Gelfoam paste alone did not induce bone nor resorb significantly in the two week implant period. This is not an unexpected result as complete resorption in 4-6 weeks in soft tissues, and liquefaction in 2-5 days under profuse bleeding conditions is the normal expectation of the Gelfoam material.

The addition of FN to the Gelfoam did not improve the rate of cell migration, adhesion or spreading significantly to improve the bone formation. It is possible that the concentration of FN used in this experiment (770 mg/mL) was not adequate, or not evenly dispersed through the paste. An alternative may be to utilize RGD peptides to increase the cell adhesivity and migration of cells to the matrix. An alternative hypothesis is that the effect of rhBMP-2 at this dosage in the rat ectopic model overwhelms any FN effect that may become detectable in a less reactive model.

The study also evaluated the dose reproducibility of the current formulation procedure. Results with labeled protein indicate that the procedure is adequate in distributing rhBMP-2 uniformly within the bulk paste. Overall, rhBMP-2 delivered in an injectable Gelfoam paste was efficacious in inducing bone in both the intramuscular and subcutaneous sites in this model.

### EXAMPLE 2

### RABBIT ULNA OSTEOTOMY STUDY

### A. Preparation of Hemostatic Gelatin Foam Matrix

A 16.7 wt % Gelfoam paste containing rhBMP-2 was prepared by hydrating Gelfoam powder (Pharmacia/Upjohn) with glutamic acid buffer (pH=4.5; 400 mg rhBMP-2) as described above for the rat ectopic study. In embodiments comprising TCP, the TCP/Gelfoam (70:30 wt ratio)containing rhBMP-2 was prepared by hydrating composite paste, TCP (sieved to 45-125 micron Gelfoam powder with glutamic acid buffer particle size) was dispersed evenly into the paste. These formulations were determined to be well-suited for injection through an 18-gauge needle while enabling the paste to maintain the cohesive consistency necessary for localization at the defect site. Additionally, a trace amount of I¹²⁵-rhBMP-2 was incorporated into the paste to allow determination of the local retention profile using scintigraphic imaging techniques.

### B. Rabbit Ulna Osteotomy Model

Bilateral mid-ulnar 1-mm osteotomy defects were created in 8 adult, male New Zealand White rabbits. One limb in each rabbit received an injection of rhBMP-2 in Gelfoam paste; the contralateral limb served as the surgical control. The Gelfoam paste (0.2 mL containing 400 µg rhBMP-2) was injected via syringe into and around the defect using an 18 gauge needle. After 4 weeks, rabbits were sacrificed and limbs excised. Callus formation was determined using peripheral quantitative computed tomography (pQCT). Biomechanical testing was performed by embedding limbs and testing to failure in torsion using a servohydraulic materials testing system (Model 8500, Instron, Corp.). Histology was performed on representative samples and sections stained with Goldner's trichrome.

In the Gelfoam/TCP study bilateral mid-ulnar 1-mm osteotomy defects were created in 24 adult, male New Zealand White rabbits (8 rabbits per group). One limb in each rabbit received an injection of rhBMP-2/carrier; the contralateral limb served as the surgical control. Each carrier or buffer (0.15 mL containing 100 µg rhBMP-2) was injected via syringe into and around the defect. In vivo biodistribution of I¹²⁵-rhBMP-2 was quantified using gamma radiography. After four weeks in vivo biodistribution and efficacy was tested as described above.

### C. Retention of I¹²⁵-rhBMP-2 in the Rabbit Osteotomy Model

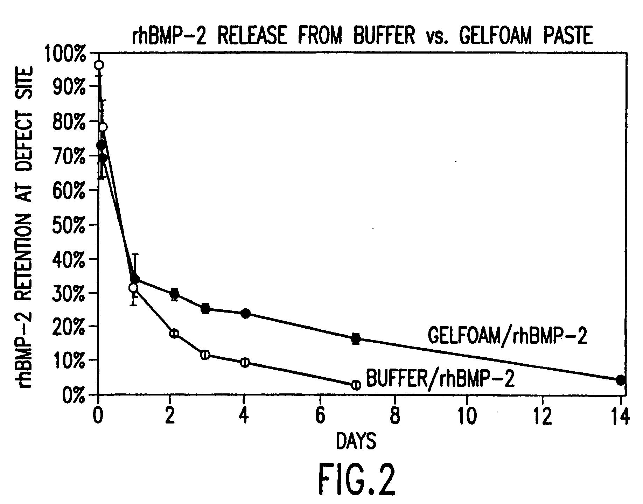

Retention of I¹²⁵-rhBMP-2 post-injection in the area of the defect (Fig. 2), was compared to rhBMP-2 delivered in a buffer vehicle for 3 rabbits. The mean residence time (MRT) was 4.43 days and 1.72 days for rhBMP-2 delivered in Gelfoam and buffer respectively. Accordingly, the rhBMP-2 half-life (t1/2) was 3.44 days and 1.11 days for Gelfoam and buffer, respectively. After 7 days, rhBMP-2 delivered in buffer was no longer detectable; however, rhBMP-2 delivered in Gelfoam was detectable at 14 days post-injection.

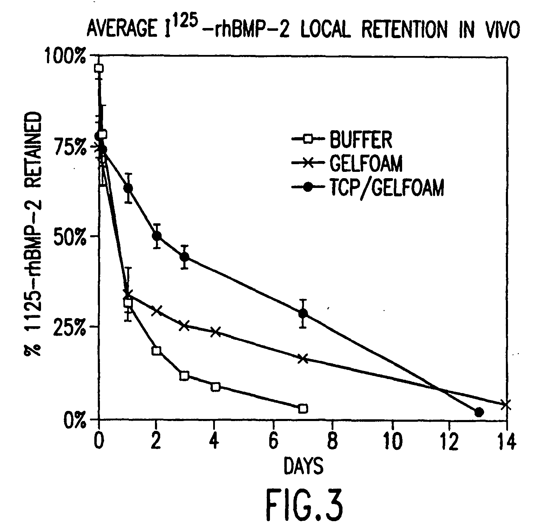

Retention of I¹²⁵-rhBMP-2 in Gelfoam and TCP/Gelfoam pastes post-injection in the area of the defect (Fig. 3), were compared to rhBMP-2 delivered in the buffer vehicle for 3 rabbits/group. The mean residence time (MRT) was 4.4 days, 5.9 days and 1.7 days for rhBMP-2 delivered in Gelfoam, TCP/Gelfoam and buffer respectively. Accordingly, the rhBMP-2 half-life (t1/2) was 3.4 days, 4.1 days and 1.1 days for Gelfoam, TCP/Gelfoam and buffer, respectively. After 7 days, rhBMP-2 delivered in buffer was no longer detectable at the site; however, rhBMP-2 delivered in Gelfoam or TCP/Gelfoam paste was detectable at 13-14 days post-injection.

### D. Biomechanical and Callus Area Assessment

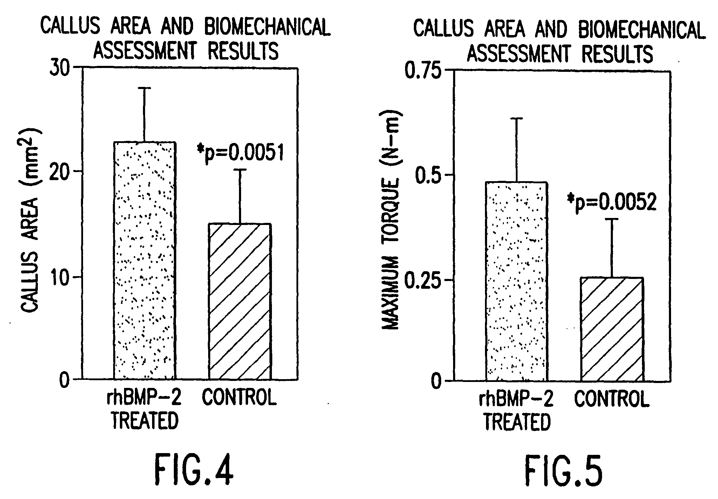

16 limbs (8 control and 8 rhBMP-2 treated) were evaluated for callus area and biomechanical strength (maximum torque). A paired t-test determined that both callus area and biomechanical strength were greater in the rhBMP-2 treated limbs than in the control limbs (Fig. 4, 5). The mean % difference between treated and control groups was 51% and 84% for callus area and maximum torque respectively.

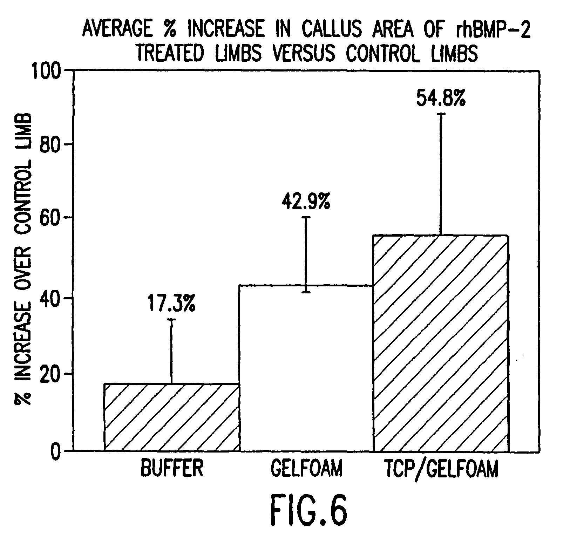

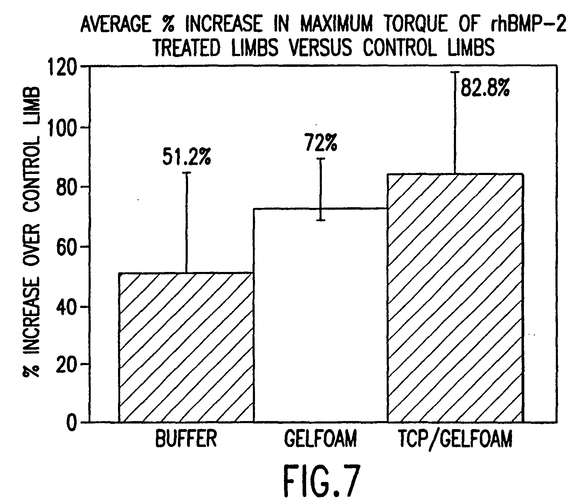

In the Gelfoam/TCP study, a paired t-test determined that both callus area and biomechanical strength were significantly greater in the rhBMP-2 treated limbs than in the control limbs for Gelfoam paste and TCP/Gelfoam paste (p<0.05). However, for rhBMP-2 delivered in buffer vehicle, the treated limbs were not statistically different than the control limb group. The mean % increase above the control was calculated for callus area and maximum torque (Figs 6-7). The % increase in callus area and maximum torque were both highest for the TCP/Gelfoam group and lowest for delivery of rhBMP-2 in buffer.

### E. Histology Assessment

Histological evaluation of 3 representative pairs of limbs in the Gelfoam study showed that the defect was 100% bridged with bony callus at the time of sacrifice (4 weeks) for the rhBMP-2-treated limbs whereas the defect was only partially-bridged for the untreated control limbs. This observation was confirmed by radiographs taken at 4 weeks.

Histology evaluation of representative limbs in the Gelfoam/TCP study showed that the defect was 100% bridged with callus for the Gelfoam and TCP/Gelfoam rhBMP-2-treated limbs whereas the defect was only partially-bridged for the untreated control and buffer limbs.

The foregoing descriptions detail presently preferred embodiments of the present invention. Numerous modifications and variations in practice thereof are expected to occur to those skilled in the art upon consideration of these descriptions. Those modifications are believed to be encompassed within the claims appended hereto.

## Claims

1. A composition for injectable delivery of osteogenic proteins comprising a pharmaceutically acceptable admixture comprising.
(a) an osteogenic protein; and
(b) a hemostatic gelatin foam paste.

2. The composition of claim 1 further comprising tricalcium phosphate.

3. The composition of claim 1 wherein the osteogenic protein is selected from the group consisting of members of the BMP family.

4. The composition of claim 3 wherein the osteogenic protein is a recombinant BMP protein.

5. The composition of claim 3 wherein the osteogenic protein is BMP-2.

6. The composition of claim 3 wherein the osteogenic protein is human BMP-2.

7. The composition of claim 3 wherein the osteogenic protein is recombinant human BMP-2.

8. A composition for injectable delivery of osteogenic proteins admixture comprising
(a) BMP-2;
(b) Hemostatic gelatin foam; and
(c) Tricalcium phosphate.

9. The composition of claim 1 wherein the osteogenic protein is OP-1.

10. A composition providing an injectable matrix for osteogenic proteins comprising a hemostatic gelatin foam paste.

11. The composition according to any one of claims 1 to 8, comprising porous microparticles and one or more antibiotics.

12. The composition of claim 11, comprising a protein sequesting agent selected from alignate and cellulosics

13. A method for making a hemostatic gelatin foam paste suitable for injecting osteogenic protein, said method comprising hydration of gelfoam powder with glutamic acid buffer.
